# EUROPEAN PATENT APPLICATION

(11) **EP 2 098 511 A1**
(43) Date of publication of application: **09.09.2009**
(21) Application number: 08152473.8
(22) Date of filing: 07.03.2008
(51) Int. Cl.: C07D 217/04, C07D 295/04

(54) **Process for preparing compounds containing a hydronaphtalene structure with an unsymmetrically substituted benzene ring**

(71) Applicant: Solvias AG, 4057 Basel (CH)
(72) Inventor: The designation of the inventor has not yet been filed
(74) Representative: Savatier, Yves

(57) **Abstract**

Process for preparing a compound according to formula (I) or (Ia) in a high enantiomeric excess, 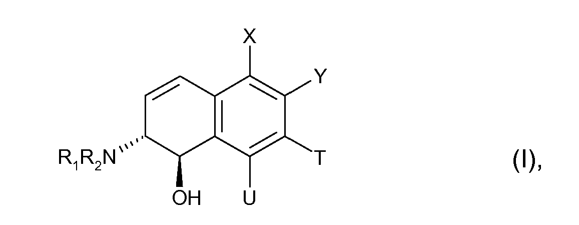 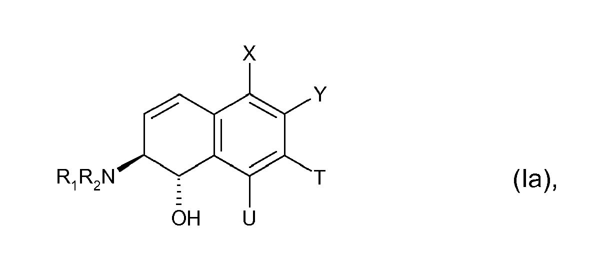 the process comprising
reacting a nucleophile of formula R₁NHR₂ with a chiral compound of formula (II),
in the form of a racemate, 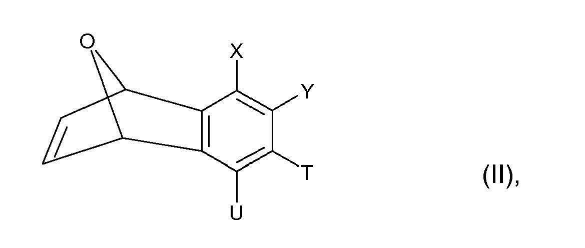 wherein R₁, R₂, X, Y, T and U are defined as above,
in the presence of a rhodium-based catalytic system which comprises a chiral diphosphine ligand.
New compounds made by that process. Use of that process or those compounds for preparing active pharmaceutical ingredients (APIs).

## Description

The present invention relates to a new process for chemically synthesizing compounds containing a hydronaphthalene structure with an unsymmetrically substituted benzene ring and the compounds made by that process. The invention further concerns the use of that process or those compounds for preparing active pharmaceutical ingredients (APIs).

The hydronaphthalene structure with an unsymmetrically substituted benzene ring can be found in many natural and synthetic products, possessing a wide range of biological activities (see e. g. Saito M. et al., J. Med. Chem. 1980, 23, 1364; Freemann J. P. et al., J. Med. Chem. 1991, 34, 1391; Jones J. H. et al. J. Med. Chem. 1984, 27, 1607; New J. S. et al. J. Med. Chem. 1984, 27, 1508; Wrick S.D.J. Med. Chem. 1993, 36, 2542; Johnansson A. M. et la., J. Med. Chem. 1987, 30, 602; Snyder S. E. J. Med. Chem. 1995, 38, 2395; Perrone R. J. Med. Chem. 1995, 38, 942), . These products include a large number of APIs that are currently marketed or under development, in particular the following APIs:
Rotigotine ((6S)-5,6,7,8-tetrahydro-6-[propyl[2-(2-thienyl)ethyl]amino]-1-naphthalenol / WO 88/08702, WO 2005/09425): 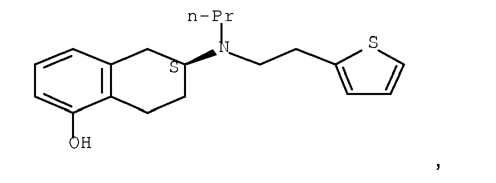
Amibegron ([[(7S)-7-[[(2R)-2-(3-chlorophenyl)-2-hydroxyethyl]amino]-5,6,7,8-tetrahydro-2-naphthalenyl]oxy]-acetic acid ethyl ester / EP-303545): 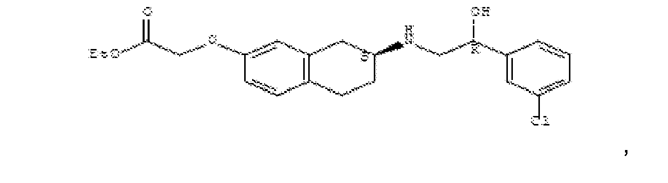
Naxagolide ((4aR,10bR)-3,4,4a,5,6,10b-hexahydro-4-propyl-2H-Naphtho[1,2-b]-1,4-oxazin-9-ol / EP-80115): 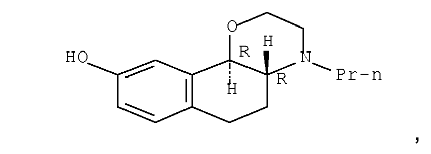
S-32504 ((4aR,10bR)-3,4,4a,5,6,10b-hexahydro-4-propyl-2H-Naphth[1,2-b]-1,4-oxazine-9-carboxamide, EP-899267): 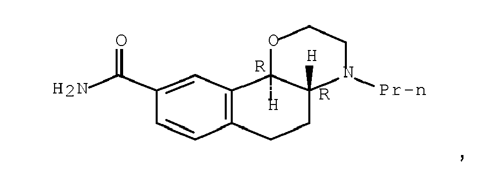
LY 274600 ((S)-1,2,3,4-tetrahydro-8-(methylthio)-N,N-dipropyl-2-naphthalenamine, EP-385658): 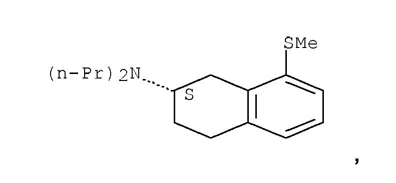
NAS-438 (4-(4-morpholinyl)-N-[(2R)-1,2,3,4-tetrahydro-8-(4-methyl-1-piperazinyl)-2-naphthalenyl]-benzamide, WO-97/34883): 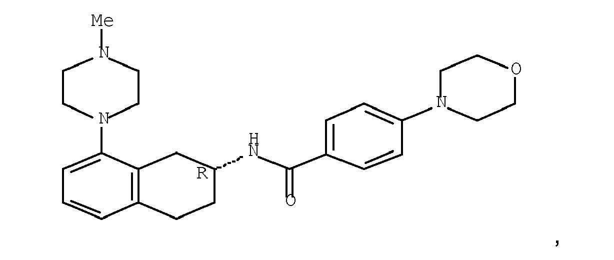
AJ-76 ((1S,2R)-1,2,3,4-tetrahydro-5-methoxy-1-methyl-N,N-dipropyl-2-naphthalenamine, EP-64964): 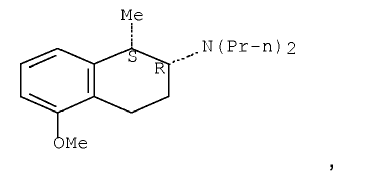
KUL-1248 ([R-(R*,S*)]-N,N-dimethyl-2-[[5,6,7,8-tetrahydro-7-[[2-hydroxy-2-(4-hydroxyphenyl)ethyl]amino]-2-naphthalenyl]oxy]-acetamide, WO-97/38970): 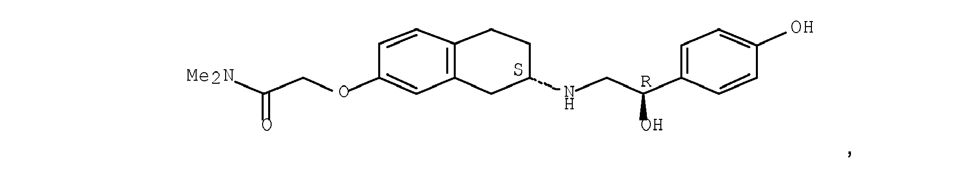
Bedoradrine / KUR-1246 (N,N-dimethyl-2-[[(7S)-5,6,7,8-tetrahydro-7-[[(2R)-2-hydroxy-2-[4-hydroxy-3-(2-hydroxyethyl)phenyl]ethyl]amino]-2-naphthalenyl]oxy]-acetamide, WO-97/30023): 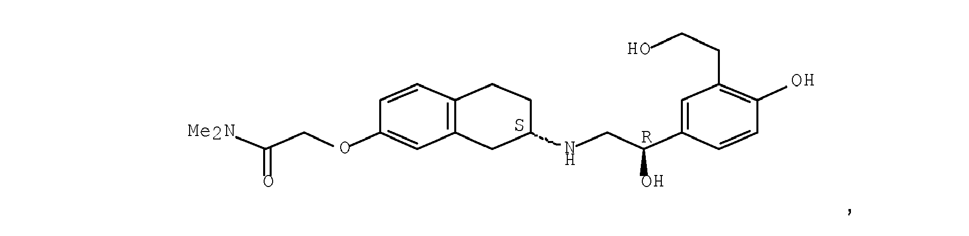
AR A000002 (4-(4-morpholinyl)-N-[(2R)-1,2,3,4-tetrahydro-5-methyl-8-(4-methyl-1-piperazinyl)-2-naphthalenyl]-benzamide, WO-99/05134): 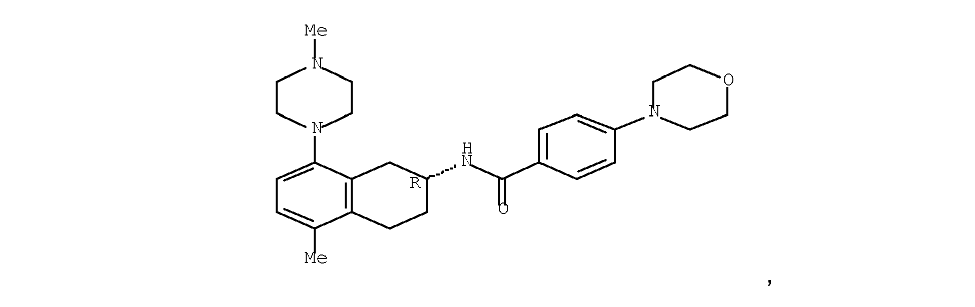 Terotroban (6-[[(4-chlorophenyl)sulfonyl]amino]-5,6,7,8-tetrahydro-2-methyl-1-naphthalenepropanoic acid, EP-688741): 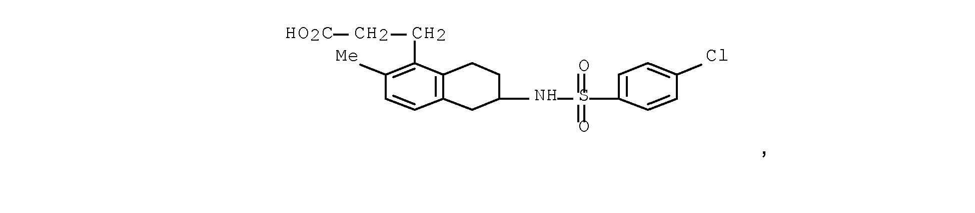
CHF-1024 (5,6,7,8-tetrahydro-6-(methylamino)-1,2-naphthalenediol, US-4163063): 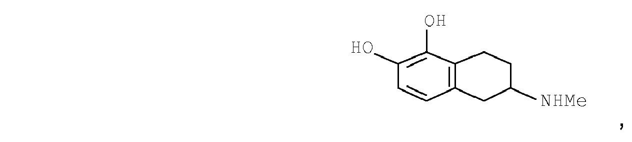 and
NOLOMIROLE / CHF-1 035 (2-methyl-propanoic acid 1,1'-[5,6,7,8-tetrahydro-6-(methylamino)-1,2-naphthalenediyl] ester, GB-2123410):

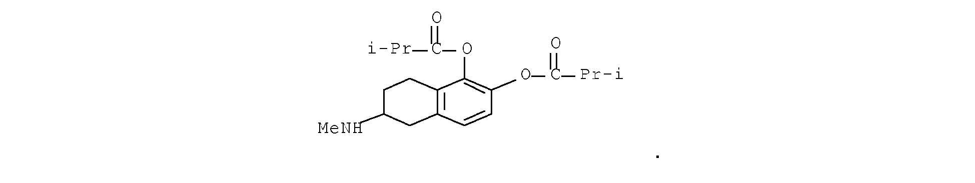

Given the large number of pharmaceutically useful compounds which contain a functionalized hydronaphthalene structure with an unsymmetrically substituted benzene ring, new methodology for producing such a hydronaphthalene skeleton with the right stereochemistry would clearly be of value.

WO 01/30734 discloses a process for synthesizing compounds containing a hydronaphthalene structure with a symmetrically substituted benzene ring, based on a rhodium catalyzed ring opening reaction of oxabenzonorbornadienes or azabicyclic compounds. This process involves producing a chiral dihydronaphthalene with a symmetrically substituted benzene ring of formula: 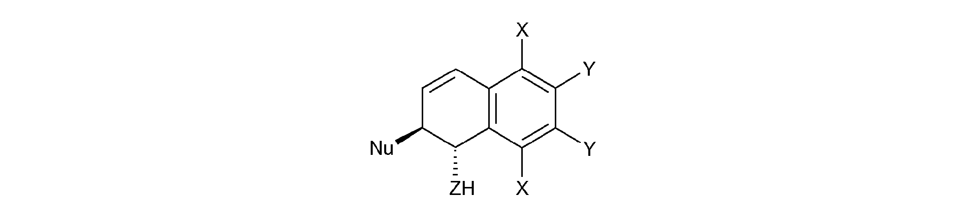 by reacting a nucleophile Nu-H, which may be a nitrogen nucleophile, a carboxylate nucleophile, a carbon nucleophile, an alcohol or phenol nucleophile, with an achiral compound of the following formula: 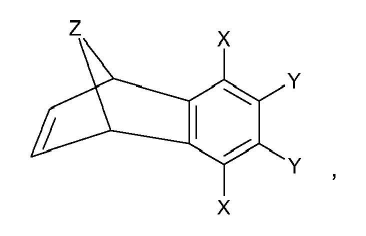 where X and Y are independently selected from the group consisting of H; NH₂; F; Cl; Br; a C₁-C₃ alkyl; and a C₁-C₃ alkoxy; or wherein the combination XY or YY together form a C₃-C₆ carbocyclic ring or a C₃-C₆ heterocyclic ring containing one or more heteroatoms selected from the group consisting of: O; N; and S; and Z is selected from O or NRₐ, wherein Rₐ is selected from
(i) a straight or branched C₁-C₆ alkyl,
(ii) phenyl,
(iii) OCOR_{b}, wherein R_{b} is a straight or branched C₁-C₆ alkyl,
(iv) SO₂-R_{c}, wherein R_{c} is unsubstituted phenyl or a phenyl substituted with a C₁-C₃ alkyl or NO₂, and
(v) SO₂-(CH₂)_{q}-Si(CH₃)₃ wherein q is 1-3,
in the presence of a catalyst comprising a metal complex of formula [Rh(COD)Cl]₂ and a phosphine ligand.

When the phosphine ligand is a chiral ligand such as (R)-(S)-BPPFA or (R)-(S)-PPF-P^{t}Bu₂, the chiral dihydronaphthalene is described to be obtained with a high enantiomeric excess.

M. Lautens et al., J. Org. Chem. 2002, 67, 8043-8053, report ring-opening of chiral oxabenzonorbornadienes with an unsymmetrically substituted benzene ring by reaction with various nucleophiles in the presence of a catalyst comprising a metal complex of formula [Rh(COD)Cl]₂ and achiral diphosphine ligand DPPF. Potentially two regioisomers **A** and **B** could be formed. It was reported that the regioselectivity of the ring-opening is highly influenced by the electronic nature of the substituents on the benzene ring of the oxabenzonorbornadiene. For example, with electron-donating substituents such as OMe and N(Me)Ph on the benzene ring of the oxabenzonorbornadiene and using MeOH as the nucleophile regioselectivities of up to >25:1 in favor of **A** were observed.

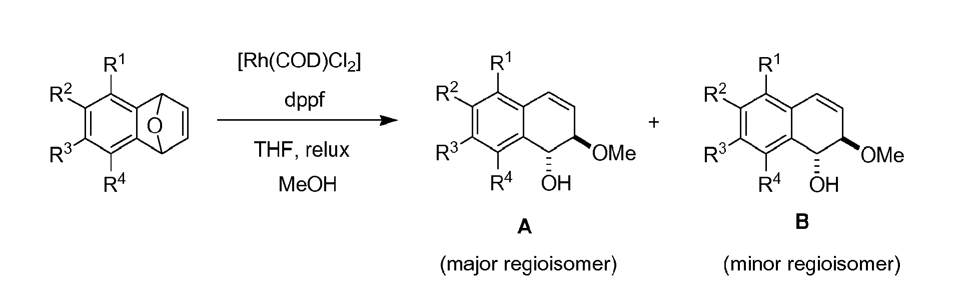

| R¹ | R² | R³ | R⁴ | Yield **A** + **B,** % | Ratio, **A:B** |
|---|---|---|---|---|---|
| OMe | H | H | H | 81 | 3.5:1 |
| H | H | OMe | H | 89 | 12:1 |
| OMe | H | OMe | H | 80 | >25:1 |
| H | H | N(Me)Ph | H | 56 | >25:1 |

The present invention is based on the unexpected discovery that when performing the rhodium catalyzed ring-opening of chiral oxabenzonorbornadienes with an unsymmetrically substituted benzene ring (containing electron-donating substituents) in the presence of a rhodium-based catalytic system comprising an enantiopure chiral diphosphine ligand, the enantiopure Rh (I) chiral diphosphine ligand complex overrides the electronic bias of the substrate resulting in formation of two regioisomers both in high enantiomeric excess.

The invention thus relates to a process for preparing a compound according to formula (I) or (Ia) in a high enantiomeric excess, 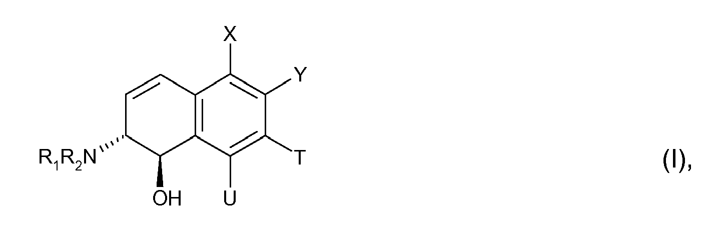 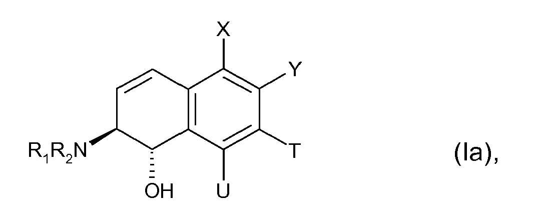 where each of X, Y, U and T is independently selected from the group consisting of H, F, Cl, Br, I, CN, C₁-C₃ alkyl which is possibly substituted by one or more halogen atoms, C₆-C₁₂ aralkyl, C₄-C₈ heteroaralkyl, C₁-C₃ alkoxy, C₆-C₁₂ aralkyloxy, C₁-C₃ alkylthio, an amino group NR₃R₄, a carboxylic acid amido or ester group (CH₂)ₓ-CO-E, x being 1 or 0 and E being OR₅ or NR₆R₇, or a group -OCO-R₅, or X and Y, Y and T, or T and U together represent a C₅-C₈ carbocyclic ring or C₃-C₆ heterocyclic ring containing one or more heteroatoms selected from the group consisting of O, N and S, which may be substituted by one or more C₁-C₃ alkyl, each of R₃ and R₄ is independently selected from the group consisting of C₁-C₁₀ alkyl, C₅-C₈ cycloalkyl, C₆-C₁₂-aralkyl, or R₃ and R₄ together represent -(CH₂)ₙ, n being 3, 4 or 5, -CH₂-CH₂-O-CH₂-CH₂- or -CH₂-CH₂-NR₈-CH₂-CH₂-, R₅ represents C₁-C₁₀ alkyl, C₅-C₈ cycloalkyl or C₆-C₁₂-aralkyl, each of R₆ and R₇ is independently selected from the group consisting of C₁-C₁₀ alkyl, C₅-C₈ cycloalkyl, C₆-C₁₂-aralkyl, or R₆ and R₇ together represent -(CH₂)ₙ, n being 3, 4 or 5, -CH₂-CH₂-O-CH₂-CH₂- or -CH₂-CH₂-NR₈-CH₂-CH₂-, R₈ represents C₁-C₃ alkyl, with the proviso that Y is different from T, and / or X is different from U (the benzene being thus unsymmetrically substituted), and each of R₁ and R₂ is independently selected from the group consisting of H, a C₁-C₁₀ carbon residue which may contain 1 or more heteroatoms selected from O, N and S, or R₁ and R₂ together represent -(CH₂)ₙ, n being 3, 4 or 5, -CH₂-CH₂-O-CH₂-CH₂-, -CH₂-CH₂-NR₈-CH₂-CH₂- or -CO-R₉-CO-, and R₉ represents 1 ,2-phenylene, ethylene, ethenylene, which may be substituted by one or two C₁-C₃ alkyl, the process comprising reacting a nucleophile of formula R₁NHR₂ with a chiral compound of formula (II), in the form of a racemate, 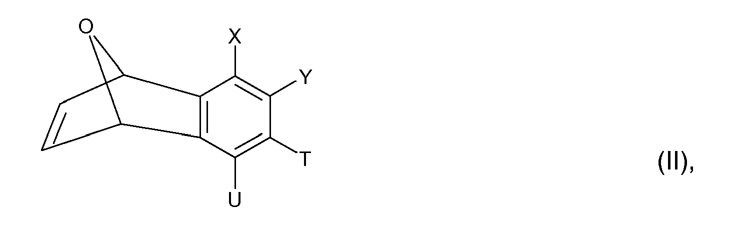 wherein R₁, R₂, X, Y, T and U are defined as above, in the presence of a rhodium-based catalytic system which comprises a chiral diphosphine ligand.

The term "in a high enantiomeric excess" here means an enantiomeric excess (ee) of at least 90 %, preferably at least 95 %, in particular at least 98 %, most preferably at least 99 %.

In formulae (I) and (Ia), typically:
- C₁-C₃ alkyl possibly substituted by one or more halogen atoms is methyl, ethyl, n-propyl or CF₃
- C₆-C₁₂ aralkyl is benzyl or phenethyl,
- C₄-C₈ heteroraralkyl is pyridinomethyl or furanomethyl
- C₁-C₃ alkoxy is methoxy, ethoxy or *n*-propyloxy,
- C₆-C₁₂ aralkyloxy is benzyloxy or phenethyloxy,
- C₁-C₃ alkylthio is methylthio, ethylthio or *n*-propylthio,
- C₅-C₈ cycloalkyl is cyclohexyl,
- group -OCO-R₅ is -OCO-*i*-propyl,
- ester group (CH₂)ₓ-CO-OR₅ is CH₂-COCH₃ or CH₂-COC₂H₅ ,
- amido group (CH₂)ₓ-CO-NR₆R₇ is CH₂CO-N(CH₃)₂ or CH₂CO-N(C₂H₅)₂, and
- amino group NR₃R₄ is N-(benzyl)₂ or N-methyl-piperazine.

Generally, in the above process another major product is produced, namely the regioisomer of the compound of formula (I) or (Ia), which is a compound according to formula (III) or (IIIa) and is also in a high enantiomeric excess:

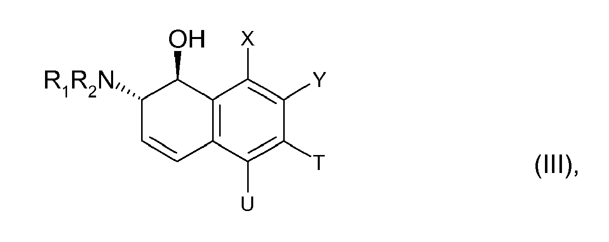

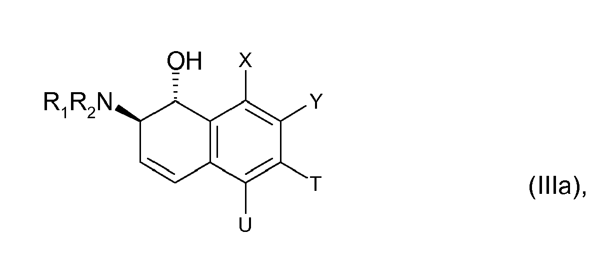

Usually the above process comprises a subsequent step of separating or isolating the compound of formula (I) or (Ia) in a high enantiomeric excess from the other components of the reaction mixture, notably the above regioisomer, by an appropriate method involving e.g. chromatography, fractional crystallisation and/or distillation. A fractional crystallisation method, if possible, is for economic reasons a method of choice.

If the compound of formula (I) or (Ia) in a high enantiomeric excess is an API, it will be isolated to a high degree of purity compatible with its use in the preparation of a medicament.

Generally the compound of formula (I) or (Ia) in a high enantiomeric excess is an intermediate to a desired API. That compound is then separated or isolated from the reaction mixture to a degree that it can be further reacted in the synthesis of the API without interference from the other components of the reaction mixture. Usually that compound will be separated from its regioisomer, which may show a similar reactivity in subsequent reactions.

The rhodium-based catalytic system may comprise a rhodium (I) complex such as [Rh(COD)Cl]₂, [Rh(CO)₂Cl]₂ or Rh(COD)₂OTf. Better yields and enantioselectivities are generally obtained when the rhodium-based catalytic system produces in situ an iodide or bromide ligand, such as e.g. Rh(COD)₂I or Rh(COD)₂Br. Such a catalytic system may for example comprise Rh(COD)₂OTf in combination with a nucleophile such as Bu₄NI, NH₄I or Bu₄NBr.

Preferably the rhodium-based catalytic system is one that produces in situ a Rh(COD)₂ iodide or bromide.

Suitable chiral diphosphine ligands are for example the Josiphos diphosphine ligands described in EP-564406, EP-646590 and EP-612758. Particularly interesting such Josiphos ligands (disclosed in EP564406) are
- (R)-(S)-PPF-P^{t}Bu₂ ((R)-1-{(S)-2-diphenylphosphinoferrocenyl)ethyl-di-tert-bultylphosphine), and
- (S)-(R)-PPF-P^{t}Bu₂ ((S)-1-{(R)-2-diphenylphosphinoferrocenyl)ethyl-di-tert-bultylphosphine).

Other interesting chiral diphosphine ligands are chiral analogues of DPPF and (R)-(S)-BPPFA and (S)-(R)-BPPFA.

Preferably the chiral diphosphine ligand is selected from the group consisting of (R)-(S)-PPF-P^{t}Bu₂, (S)-(R)-PPF-P^{t}Bu₂, (R)-(S)-BPPFA, (S)-(R)-BPPFA and a chiral analogue of DPPF.

Preferably the nucleophile of formula R₁NHR₂ is chosen so that the compound of formula (I) or (Ia) is a crystalline product, apt to be separated or isolated by fractional crystallisation.

The nucleophile of formula R₁NHR₂ may be an acyclic secondary amine, such as e.g. dipropyl amine, or a primary amine (one of R₁ and R₂ being H), such as e.g. propylamine. In the latter case the nitrogen atom of the amino group R₁R₂N- is available for a further substitution, if necessary.

The nucleophile of formula R₁NHR₂ may comprise a ring selected from a phtalimide ring, a piperidine ring, a pyrrolidine ring, an indole ring and a tetrahydroquinoline ring, the ring being optionally substituted at one or more positions with a group selected from a C₁-C₃ alkyl; a C₁-C₃ alkoxy; Cl; F; NO₂; and CF₃.

In preferred reactions the nucleophile of formula R₁NHR₂ is selected from the group consisting of phtalimide, piperidine, pyrrolidine, indole, tetrahydroquinoline, dibenzylamine, benzylamine, p-methoxybenzylamine , dipropylamine and propylamine.

In particularly preferred reactions the nucleophile of formula R₁NHR₂ is selected from the group consisting of phtalimide, dibenzylamine, benzylamine, dipropylamine and propylamine.

The process may be carried out using THF or a mixture of THF and DMF as solvent under an inert atmosphere, preferably of nitrogen.

The reaction temperature should be at least 60 °C, preferably about 80 °C.

The invention also relates to the new chiral compounds which are obtained in a high enantiomeric excess by the above process.

The invention thus concerns a compound according to formula (I) or (Ia) in a high enantiomeric excess, 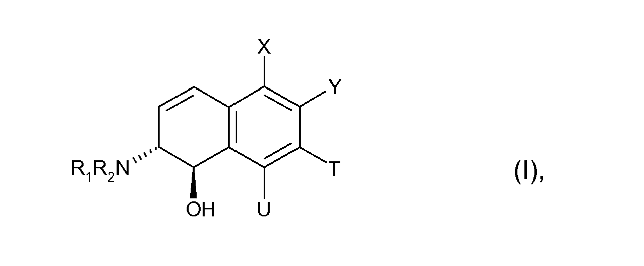 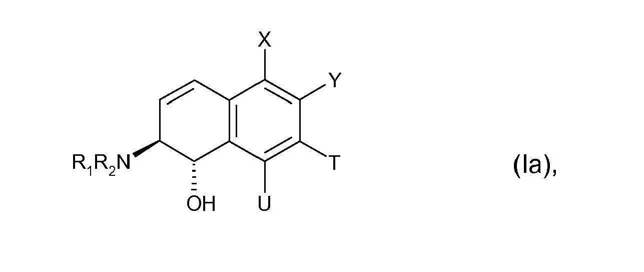 each of X, Y, U and T is independently selected from the group consisting of H, F, Cl, Br, I, CN, C₁-C₃ alkyl which is possibly substituted by one or more halogen atoms, C₆-C₁₂ aralkyl, C₄-C₈ heteroaralkyl, C₁-C₃ alkoxy, C₆-C₁₂ aralkyloxy, C₁-C₃ alkylthio, an amino group NR₃R₄, a carboxylic acid amido or ester group (CH₂)ₓ-CO-E, x being 1 or 0 and E being OR₅ or NR₆R₇, or a group - OCO-R₅, or X and Y, Y and T, or T and U together represent a C₅-C₈ carbocyclic ring or C₃-C₆ heterocyclic ring containing one or more heteroatoms selected from the group consisting of O, N and S, which may be substituted by one or more C₁-C₃ alkyl, each of R₃ and R₄ is independently selected from the group consisting of C₁-C₁₀ alkyl, C₅-C₈ cycloalkyl, C₆-C₁₂-aralkyl, or R₃ and R₄ together represent -(CH₂)ₙ, n being 3, 4 or 5, -CH₂-CH₂-O-CH₂-CH₂- or -CH₂-CH₂-NR₈-CH₂-CH₂-, R₅ represents C₁-C₁₀ alkyl, C₅-C₈ cycloalkyl or C₆-C₁₂-aralkyl, each of R₆ and R₇ is independently selected from the group consisting of C₁-C₁₀ alkyl, C₅-C₈ cycloalkyl, C₆-C₁₂-aralkyl, or R₆ and R₇ together represent -(CH₂)ₙ, n being 3, 4 or 5, -CH₂-CH₂-O-CH₂-CH₂- or -CH₂-CH₂-NR₈-CH₂-CH₂-, R₈ represents C₁-C₃ alkyl, with the proviso that Y is different from T, and / or X is different from U, and each of R₁ and R₂ is independently selected from the group consisting of H, a C₁-C₁₀ carbon residue which may contain 1 or more heteroatoms selected from O, N and S, or R₁ and R₂ together represent -(CH₂)ₙ, n being 3, 4 or 5, -CH₂-CH₂-O-CH₂-CH₂-, -CH₂-CH₂-NR₈-CH₂-CH₂- or -CO-R₉-CO-, and R₉ represents 1,2-phenylene, ethylene, ethenylene, which may be substituted by one or two C₁-C₃ alkyl.

In an interesting embodiment one of R₁ and R₂ is H. The nitrogen atom of R₁R₂N- is then available for further substitution, if necessary.

Examples of interesting compounds of formula (I) or (Ia) are:
- 2-[(1S_{,}2S)-5-(benzyloxy)-1-hydroxy-1_{,}2-dihydronaphthalen-2-yl]isoindoline-1_{,}3-dione (intermediate for Rotigotine),
- 2-[(1S,2S)-7-(benzyloxy)-1-hydroxy-1,2-dihydronaphthalen-2-yl]isoindoline-1,3-dione (intermediate for Amibegron),
- (1R,2R)-7-(benzyloxy)-2-(benzylisopropylamino)-1_{,}2-dihydronaphthalen-1-ol (intermediate for Naxagolide)
- (1*R*,2*R*)-2-(dipropylamino)-1,2-dihydro-5-methoxynaphthalen-1-ol (intermediate for AJ-76),
- 2-[(1*S*,2*S*)-7-(benzyloxy)-1,2-dihydro-1-hydroxynaphthalen-2-yl]isoindoline-1,3-dione (intermediate for KUL-1248), and
- 2-[(1*S*,2*S*)-7-(benzyloxy)-1,2-dihydro-1-hydroxynaphthalen-2-yl]isoindoline-1,3-dione (intermediate for Bedoradrine).

When the above process is used for preparing 2-[(1S_{,}2S)-5-(benzyloxy)-1-hydroxy-1,2-dihydronaphthalen-2-yl]isoindoline-1,3-dione, the nucleophile of formula R₁NHR₂ is preferably phtalimide. A yield of 44 % of that compound with an enantiomeric excess (ee) of 98 % can then be obtained. That compound may be isolated by fractional crystallisation. The desired API Rotigotine may be prepared by four further synthetic steps: A global reduction by hydrogenation/hydrogenolysis (alkene reduction, O benzyl removal, and benzylic alcohol reduction); phthalimide removal to leave a primary amine and then two successive reductive aminations using propanal and then 2-(thiophene-2-yl)acetaldehyde.

When the above process is used for preparing 2-[(1S,2S)-7-(benzyloxy)-1-hydroxy-1,2-dihydronaphthalen-2-yl]isoindoline-1,3-dione, the nucleophile of formula R₁NHR₂ is preferably phtalimide. A yield of 55 % of that compound with an enantiomeric excess (ee) of 94 % can then be obtained. The desired API Amibegron may be prepared by four further synthetic steps. A global reduction by hydrogenation/hydrogenolysis (alkene reduction, O benzyl removal, and benzylic alcohol reduction); alkylation of the resulting phenol with ethyl 2-bromoacetate; phthalimide removal to leave a primary amine and then epoxide ring-opening using (R)-2-(3-chlorophenyl)oxirane.

The invention also concerns the use of the above process or the above compound for preparing an API, in particular an API selected from the group consisting of
- (6S)-5,6,7,8-tetrahydro-6-[propyl[2-(2-thienyl)ethyl]amino]-1-naphthalenol,
- [[(7S)-7-[[(2R)-2-(3-chlorophenyl)-2-hydroxyethyl]amino]-5,6,7,8-tetrahydro-2-naphthalenyl]oxy]-acetic acid ethyl ester,
- (4aR,10bR)-3,4,4a,5,6,10b-hexahydro-4-propyl-2H-Naphtho[1,2-b]-1,4-oxazin-9-ol,
- (4aR,10bR)-3,4,4a,5,6,10b-hexahydro-4-propyl-2H-Naphth[1,2-b]-1,4-oxazine-9-carboxamide,
- (S)-1,2,3,4-tetrahydro-8-(methylthio)-N,N-dipropyl-2-naphthalenamine,
- 4-(4-Morpholinyl)-N-[(2R)-1,2,3,4-tetrahydro-8-(4-methyl-1-piperazinyl)-2-naphthalenyl]-benzamide,
- (1S,2R)-1,2,3,4-tetrahydro-5-methoxy-1-methyl-N,N-dipropyl-2-naphthalenamine,
- [R-(R*,S*)]-N,N-dimethyl-2-[[5,6,7,8-tetrahydro-7-[[2-hydroxy-2-(4-hydroxyphenyl)ethyl]amino]-2-naphthalenyl]oxy]-acetamide,
- N,N-dimethyl-2-[[(7S)-5,6,7,8-tetrahydro-7-[[(2R)-2-hydroxy-2-[4-hydroxy-3-(2-hydroxyethyl)phenyl]ethyl]amino]-2-naphthalenyl]oxy]-acetamide,
- 4-(4-morpholinyl)-N-[(2R)-1,2,3,4-tetrahydro-5-methyl-8-(4-methyl-1-piperazinyl)-2-naphthalenyl]-benzamide,
- 6-[[(4-chlorophenyl)sulfonyl]amino]-5,6,7,8-tetrahydro-2-methyl-1-naphthalenepropanoic acid,
- 5,6,7,8-tetrahydro-6-(methylamino)-1,2-naphthalenediol, and
- 2-methyl-propanoic acid 1,1'-[5,6,7,8-tetrahydro-6-(methylamino)-1,2-naphthalenediyl] ester.

The following examples illustrate the invention.
All reactions were carried out in oven-dried or flame-dried glassware under an atmosphere of argon. DMF was distilled and stored over molecular sieves. THF was distilled from Na/benzophenone prior to use. Furan and iPr₂NH were distilled from CaH₂ prior to use. Phtalimide was recrystallized from EtOH prior to use.

### Example R1:

Preparation of (1S,2S)-5-(benzyloxy)-2-(dibenzylamino)-1,2-dihydronaphthalen-1-ol **25** (intermediate in the synthesis of Rotigotine)

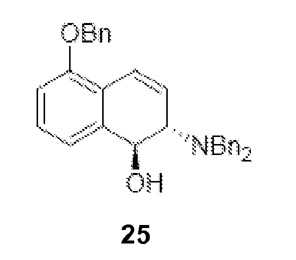

### a) Preparation of racemic 5-(benzyloxy)-I,4-dihydro-I,4-epoxynaphthalene 24

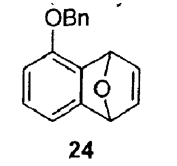

A solution of nBuLi (1.6 M in hexane; 26 mL, 41.2 mmol) was added over 3 h *via* a syringe pump to a solution of 3-(benzyloxy)-1-chlorobenzene (commercially available from Ryan Scientific and Ambinter Stock Screening Collection, 9.0 g, 41.2 mmol), furan (15 mL, 206 mmol) and diisopropylamine (0.58 mL, 4.1 mmol) in THF (200 mL) at -15 °C. After an additional 1 h the reaction was quenched with water (200 mL).

The layers were separated and the aqueous layer extracted with Et₂O (3 x 200 mL). the combined organic layers were dried (MgSO₄), and the solvent evaporated under vacuum. The residue was purified by column chromatography (silica, 100% hexane → 10% EtOAc in hexane) to give *oxabicycle* **24** (5.2 g, 50%) as a colourless oil; *R*_{f} 0.25 (25% EtOAc in hexane); IR (neat)/cm⁻¹ 3027w, 2925w, 1614m, 1596s, 1474s, 1454m, 1279s, 1179w; ₁H NMR (400 MHz) δ 7.42-7.31 (m, 5H), 7.02-6.97 (m, 2H), 6.94-6.91 (m, 2H), 6.64 (ddd, *J* = 9, 4.5, 3.5, 1H), 5.95 (br s, 1 H), 5.69 (br s , 1 H), 5.08 (s, 2H); ₁₃C NMR (100 MHz) δ 152.1, 151.6, 143.0, 142.8, 137.0, 135.7, 128.6, 128.0, 127.4, 126.9, 114.0, 112.0, 82.5, 80.1, 70.7; MS *m*/*z* (EI) 250 (M+, 5), 90 100); HRMS calculated for C₁₇H₁₄O₂ (M+) 250.0994, found 250.0991.

### b) Preparation of a mixture of (1S,2S)-5-(benzyloxy)-2-(dibenzylamino)-1,2-dihydronaphthalen-1-ol 25 and (1S,2S)-8-(benzyloxy)-2-(dibenzylamino)-1,2-dihydronaphthalen-1-ol 26, and isolation of those compounds

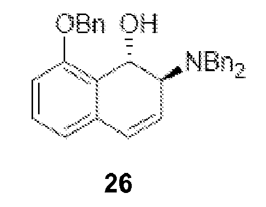

A solution of oxabicycle **24** (30 mg, 0.12 mmol) and dibenzylamine (12 µL, 0.06 mmol) in THF (0.3 mL) was added via a syringe to a solution of Rh(cod)₂OTf (rhodium-cyclooctadien-triflat, 1.4 mg, 3 µmol), (*S*,*R*)-PPF-tBu₂ (3.3 mg, 6 µmol) and Bu₄NI (9 mg, 24 µmol) in THF (0.3 mL) at RT. The reaction mixture was heated to reflux for 4 h. After cooling to RT, the reaction mixture was filtered through a pad of silica (washing with EtOAc) and the solvent was removed under vacuum.

The residue was purified by column chromatography (silica, 100% hexane → 10% EtOAc in hexane) to give (1*S*,2*S*)-5-(benzyloxy)-2-(dibenzylamino)-1,2-dihydronaphthalen-1-ol **25** (11 mg, 21 %) as a colourless oil; *R*_{f} 0.30 (25% EtOAc in hexane); IR (neat)/cm⁻¹ 3487br, 3060w, 3027w, 2925w, 1620w, 1598w, 1573m, 1494m, 1453s, 1372m, 1264s; ₁H NMR (400 MHz) δ 7.43-7.15 (m, 17H), 7.00 (dd, *J* = 10, 3, 1 H), 6.82-6.80 (m, 1 H), 6.12 (dd, *J* = 10, 2.5, 1 H), 5.06 (s, 2H), 4.97 (d, *J* = 12, 1 H), 3.97 (d, *J* = 13.5 , 2H), 3.65 (dt, *J* = 12, 2.5, 1 H), 3.59 (d, *J* = 13.5, 2H), 3.10 (s, 1 H); ₁₃C NMR (100 MHz) δ 153.8, 139.1, 138.5, 137.1, 129.0, 128.6, 128.5, 128.5, 127.9, 127.3, 127.3, 124.4, 123.8, 120.9, 117.5, 111.2, 70.3, 69.0, 62.1, 54.7; MS *m*/*z* (EI+) 447 (M+, 3), 430 (15), 357 (15), 338 (18), 252 (15), 196 (26), 91 (100); HRMS calculated for C₃₁H₂₉NO₂ (M+) 447.2198, found 447.2201.

Second to elute was (1*S*,2*S*)-8-(benzyloxy)-2-(dibenzylamino)-1,2-dihydronaphthalen-1-ol **26** (15 mg, 28%) as a colourless oil; *R*_{f} 0.24 (25% EtOAc in hexane); IR (neat)/cm⁻¹ 3487br, 3027w, 2925w, 1621w, 1598m, 1573m, 1453s, 1372m, 1264; ₁H NMR (400 MHz) δ 7.47-7.17 (m, 16H), 6.86 (dd, *J* = 9.5, 1, 1 H), 6.77, (d, *J* = 7.5, 1 H), 6.67 (dd, *J* = 9.5, 1.5, 1 H), 6.03 (ddd, *J* = 9.5, 5, 1, 1 H), 5.47 (s, 1 H), 5.22 (d, *J* = 12, 1H), 5.16 (d, *J* = 12, 1H), 3.66 (dt, *J* = 5, 1.5, 1 H), 3.56 (d, *J* = 14, 2H), 3.44 (d, *J* = 14, 2H), 2.07 (br s, 1 H); ₁₃C NMR (100 MHz) δ 155.9, 140.1, 136.9, 133.3, 129.2, 128.7, 128.7, 128.6, 128.1, 128.1, 127.3, 126.8, 126.8, 124.1, 120.4, 112.0, 70.2, 62.7, 58.6, 53.7; MS *m*/*z* (EI+) 447 (M+, 2), 196 (30), 91 (100); HRMS calculated for C₃₁H₂₉NO₂ (M+) 447.2198, found 447.2198.

The enantioselectivies were determined by chiral HPLC. 1,2-Dihydronapthalene **25** conditions: Chiralcel OD; hexane:*i*PrOH = 90:10; flow = 1.5 mL/min at RT; retention times = 10.24 min (major using (*S*,*R*)-PPF-*t*Bu₂) and 14.70 min (minor using (*S*,*R*)-PPF-*t*Bu₂). 1,2-Dihydronapthalene **26** conditions: Chiralcel OD; hexane:*i*PrOH = 90:10; flow = 1.5 mL/min at RT; retention times = 12.29 min (major using (*S*,*R*)-PPF-*t*Bu₂) and 25.30 min (minor using (*S*,*R*)-PPF-*t*Bu₂).

The above reaction conditions provided a yield of 21 % of desired (1*S*,2*S*)-5-(benzyloxy)-2-(dibenzylamino)-1,2-dihydronaphthalen-1-ol **25** with 90% ee and 28% of (1S,2*S*)-8-(benzyloxy)-2-(dibenzylamino)-1,2-dihydronaphthalen-1-ol **26** with 92% ee.

### Example R2:

Preparation of 2-[(1S,2*S*)-5-(benzyloxy)-1-hydroxy-1,2-dihydronaphthalen-2-yl]isoindoline-1,3-dione **27** (intermediate in the synthesis of Rotigotine)

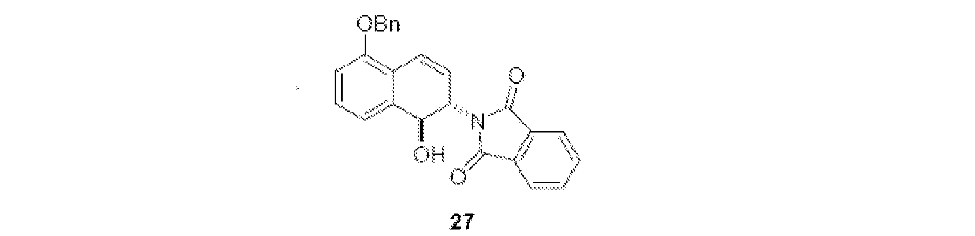

### a) Preparation of a mixture of 2-[(1S_{,}2S)-5-(benzyloxy)-1-hydroxy-1,2-dihydronaphthalen-2-yl]isoindoline-1_{,}3-dione 27 and 2-[(1S,2S)-8-(benzyloxy)-1-hydroxy-1,2-dihydronaphthalen-2-yl]isoindoline-1,3-dione 28, and isolation of those compounds

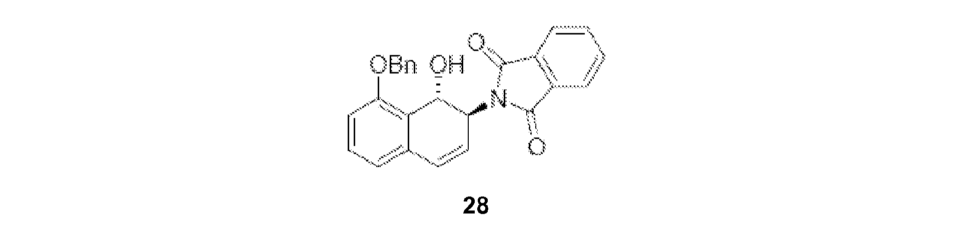

A solution of the Rh(I) complex was prepared by addition of Bu₄NI (100 mg, 0.27 mmol) to a solution of Rh(cod)₂OTf (16 mg, 34 µmol) and (*S*,*R*)-PPF-*t*Bu₂ (37 mg, 68 µmol) in THF (1.1 mL) at RT. After 5 min of stirring this solution was transferred via cannula to a solution of oxabicycle **24** (338 mg, 1.35 mmol) and phthalimide (198 mg, 1.35 mmol) in DMF (2.3 mL) and the resulting solution was heated to 95 °C for 6 h. After cooling to RT, the reaction mixture was diluted with water (20 mL) and extracted with CH₂Cl₂ (3 x 50 mL). The combined organic layers were dried (MgSO₄) and the solvent removed under vacuum [the majority of DMF was removed by gentle heating under high vacuum (0.1 mm Hg)].

The residue was purified by column chromatography (silica, 10% →20% EtOAc in hexane) to give 2-[(*1S,2S*)-8-(benzyloxy)-1-hydroxy-1,2-dihydronaphthalene-2-yl]isoindoline-1,3-dione **28** (209 mg, 39%) as a white solid; *R*_{f} 0.26 (50% EtOAc in hexane); mp 130-132 °C; [α]_{D 24} = +230 (c= 2, CHCl₃); IR (CHCl₃)/cm⁻¹ 3531 br, 3017m, 1772w, 1717s, 1387m; ₁H NMR (400 MHz) δ 7.84-7.80 (m, 2H), 7.72-7.67 (m, 2H), 7.39-7.23 (m, 6H), 6.94 (dd, *J* = 8, 0.5, 1 H), 6.85 (d, *J* = 7.5, 1 H), 6.58 (dd, *J* = 10, 2.5, 1 H), 5.82 (dd, *J* = 10, 3.5, 1 H), 5.64 (dd, *J* = 8, 1.5, 1 H), 5.33 (ddd, *J* = 8, 3.5, 2.5, 1H), 5.12 (d, *J* = 11, 1H), 5.08 (d, *J* = 11, 1 H), 3.95 (d, *J* = 1.5, 1H); ₁₃C NMR (100 MHz) δ 167.8, 156.3, 136.0, 133.9, 133.2, 132.0, 129.1, 128.8, 128.7, 128.3, 127.7, 124.9, 123.5, 123.3, 120.8, 112.2, 70.7, 68.9, 52.8; MS *m*/*z* (ESI) 420 (M+Na+, 100), 380 (90); HRMS calculated for C₂₅H₁₉NONa (M+Na+) 420.1206, found 420.1203.

Second to elute was 2-[(1*S*,2*S*)-5-(benzyloxy)-1-hydroxy-1,2-dihydronaphthalene-2-yl]isoindoline-1,3-dione **27** (236 mg, 44%) as a white solid; *R*_{f} 0.23 (50% EtOAc in hexane); mp 212-215 °C; [α]_{D 26} = +63 (c= 2.5, CHCl₃); IR (CHCl₃)/cm-1 3440br, 3018m, 1770w, 1715m, 1470m, 1389m; ₁H NMR (400 MHz) δ 7.87-7.83 (m, 2H), 7.75-7.70 (m, 2H), 7.45-7.31 (m, 5H), 7.26-7.22 (m, 2H), 7.02 (dd, *J* = 10, 3, 1 H), 6.89 (dd, *J* = 7.5, 2, 1 H), 5.87 (dd, *J* = 10, 2.5, 1 H), 5.84 (dd, *J* = 13, 9.5, 1 H), 5.13 (ddd, *J* = 13, 3, 2, 1H), 5.11 (s, 2H), 2.02 (d, *J* = 9.5, 1 H); ₁₃C NMR (100 MHz) δ 168.4, 154.1, 138.8, 136.9, 134.1, 132.0, 128.9, 128.6, 127.9, 127.3, 125.6, 123.4, 122.8, 121.6, 116.7, 111.9, 71.1, 70.4, 54.9; MS *m*/*z* (EI+) 397 (M+, 3), 250 (41), 159 (16), 91 (100); HRMS calcd for C₂₅H₁₉NO₄ (M+) 397.1314, found 397.1314.

The enantioselectivies were determined by chiral HPLC. 1,2-Dihydronapthalene **27** conditions: Chiralcel OD; hexane:*i*PrOH = 90:10; flow = 1.5 mL/min at RT; retention times = 25.10 min (minor using (*S*,*R*)-PPF-*t*Bu₂) and 39.20 min (major using (*S*,*R*)-PPF-*t*Bu₂).

1,2-Dihydronapthalene **28** conditions: Chiralpak AD; hexane:*i*PrOH = 92:8; flow = 1.5 mL/min at RT; retention times = 48.95 min (minor using (*S*,*R*)-PPF-*t*Bu₂) and 52.94 min (major using (*S*,*R*)-PPF-*t*Bu₂).

The above reaction conditions provided a yield of 44% of desired 2-[(1*S*,2*S*)-5-(benzyloxy)-1- hydroxy-1,2-dihydronaphthalene-2-yl]isoindoline-1,3-dione 27 with 98% ee and 39% yield of 2-[(*1S,2S*)-8-(benzyloxy)-1-hydroxy-1,2-dihydronaphthalene-2-yl]isoindoline-1,3-dione **28** with 99% ee.

### b) Large scale isolation procedure for 2-[(1S_{,}2S)-5-(benzyloxy)-1-hydroxy-1,2-dihydronaphthalen-2-yl]isoindoline-I_{,}3-dione 27 by recrystallization

A solution of the Rh(I) complex was prepared by addition of Bu₄NI (236 mg, 0.64 mmol) to a solution of Rh(cod)₂OTf (19 mg, 40 µmol) and (*S*,*R*)-PPF-*t*Bu₂ (87 mg, 0.16 mmol) in THF (13 mL) at RT. After 5 min of stirring this solution was transferred via cannula to a solution of oxabicycle **24** (4.0 g, 16.0 mmol) and phthalimide (2.35 g, 16.0 mmol) in DMF (27 mL) and the resulting solution was heated to 95 °C for 6 h. After cooling to RT, the reaction mixture was diluted with water (50 mL) and extracted with CH₂Cl₂ (3 x 50 mL). The combined organic layers were dried (MgSO₄) and the solvent removed under vacuum [the majority of DMF was removed by gentle heating under high vacuum (0.1 mm Hg)]. Hot MeOH (∼100 mL) was added to the resulting dark brown viscous oil, with gentle swirling until all the oil was in solution. The flask was left standing at RT for approximately 2 h. The resulting grey solid was filtered off to give 1,2-dihydronapthalene **27** (2.22 g, 35%, 97% isomerically pure by ₁H NMR). Reheating the MeOH solution and standing for a further 3 h gave a 2nd crop of 1,2-dihydronapthalene **27** (254 mg, 4%, 97% isomerically pure by ₁H NMR).

The above results show the possibility of isolating 2-[(1S,2S)-5-(benzyloxy)-1-hydroxy-1,2-dihydronaphthalen-2-yl]isoindoline-1_{,}3-dione **27** by fractional crystallization.

### Example A1:

Preparation of 2-[(1S,2S)-7-(benzyloxy)-1-hydroxy-1,2-dihydronaphthalen-2-yl]isoindoline-1,3-dione **55** (intermediate in the synthesis of Amibegron)

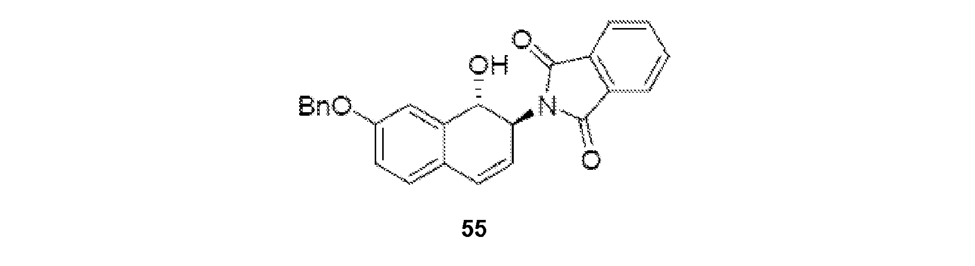

a) Preparation of racemic 6-(benzyloxy)-1,4-dihydro-1,4-epoxynaphthalene **54** 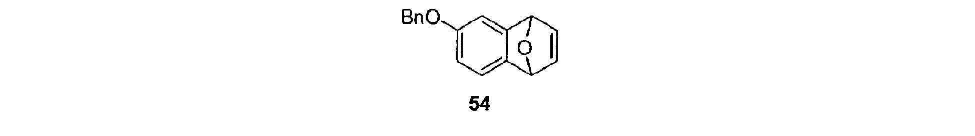 Aq. 1 M HCl (0.16 mL) was added to a solution of 3-(benzyloxy)-1-bromobenzene (commercially available from Aldrich, 4.24 g, 16.1 mmol) and NBS (2.87 g, 16.1 mmol) in acetone (16 mL) at RT. After stirring for 30 min the solvent was removed under vacuum. Hexane (20 mL) was added and the resulting white solid filtered off (succinamide). The solvent was removed under vacuum to give 4-(benzyloxy)-1,2-dibromobenzene (5.5 g). The dibromide was used in the next step without further purification. nBuLi (1.6 M in hexanes; 10.0 mL, 16.1 mmol) was added dropwise to a solution of furan (5.8 mL, 80 mmol) and 4- (benzyloxy)-1,2-dibromobenzene (5.5 g) in THF (80 mL) at -78 °C. After 30 min, the reaction was quenched with water (100 mL). After warming to RT the layers were separated and the aqueous layer was extracted with Et₂O (3 x 100 mL). The combined organic layers were dried (MgSO₄), and the solvent evaporated under vacuum.

The residue was purified by column chromatography (silica, 100% hexane → 20% EtOAc in hexane) to give 6-(benzyloxy)-1,4-dihydro-1,4-epoxynaphthalene **54** (2.01 g, 50%, over 2 steps) as a colourless oil, which solidified on standing; *R*_{f} 0.35 (25% EtOAc in hexane); mp = 75-77 °C; IR (neat)/cm⁻¹ 3091w, 1599m, 1455s, 1283m, 1222s, 1086m; ₁H NMR (400 MHz) δ 7.41-7.28 (m, 5H), 7.10 (d, *J*= 8, 1 H), 7.01 (dd, *J* = 5.5, 1.5, 1 H), 6.98-6.96 (m, 2H), 6.48 (dd, *J* = 8, 2.5, 1 H), 5.65 (dd, *J* = 8, 1.5, 2H), 5.00 (s, 2H); ₁₃C NMR (100 MHz) δ 156.6, 151.0, 143.4, 142.2, 140.7, 137.0, 128.5, 127.9, 127.4, 120.3, 110.3, 108.4, 82.4, 82.0, 70.4; MS *m*/*z* (EI+) 250 (M+, 15), 91 (100); HRMS calcd for C₁₇H₁₄O₂ (M+) 250.0994, found 250.0999.

### b) Preparation of a mixture of 2-[(1S,2S)-7-(benzyloxy)-1-hydroxy-1,2-dihydronaphthalene-2-yl]isoindoline-1,3-dione 55 and 2-[(1S,2S)-6-(benzyloxy)-1-hydroxy-1,2-dihydronaphthalene-2-yl]isoindoline-1,3-dione 56, and isolation of those compounds

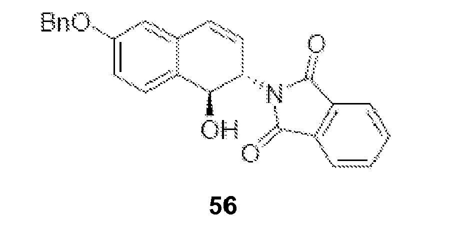

A solution of the Rh(I) complex was prepared by addition of Bu₄NI (15 mg, 40 µmol) to a solution of Rh(cod)₂OTf (1.2 mg, 2.5 µmol) and (*S*,*R*)-PPF-*t*Bu₂ (5.4 mg, 10 µmol) in THF (0.8 mL) at RT. This solution was transferred via cannula to a solution of oxabicycle **54** (250 mg, 1.0 mmol) and phthalimide (147 mg, 1.0 mmol) in DMF (1.6 mL) and the resulting solution was heated to 95 °C for 6 h. After cooling to rt, the reaction mixture was diluted with water (20 mL) and extracted with CH₂Cl₂ (3 x 30 mL). The combined organic layers were dried (MgSO₄) and the solvent removed under vacuum [the majority of DMF was removed by gentle heating under high vacuum (0.1 mm Hg)].

The residue was purified by column chromatography (silica, 10% → 20% EtOAc in hexane) to give 2-[(1S,2S)-7-(benzyloxy)-1-hydroxy-1,2-dihydronaphthalene-2-yl]isoindoline-1,3-dione **55** (218 mg, 55%) as a white solid; *R*_{f} 0.26 (40% EtOAc in hexane); mp 198-200 °C; [α]D²⁵ = +43 (c = 5.3, CHCl₃); IR (CHCl₃)/cm⁻¹ 3531 br, 3015m, 1772w, 1717s, 1380m; ₁H NMR (400 MHz) δ 7.84-7.79 (m, 2H), 7.71-7.66 (m, 2H), 7.44-7.25 (m, 6H), 7.02 (d, *J* = 8, 1 H), 6.82 (dd, *J* = 8, 2.5, 1 H), 6.48 (dd, *J* = 10, 3, 1 H), 5.72 (dd, *J* = 10, 2.5, 1 H), 5.46 (dd, *J* = 12.5, 9.5, 1 H), 5.16 (ddd, *J* = 13, 3, 2.5, 1 H), 5.07 (s, 2H), 2.37 (d, *J* = 9.5, 1 H); ₁₃C NMR (100 MHz) δ 168.5, 158.9, 139.0, 136.8, 134.0, 131.9, 128.5, 128.0, 128.0, 127.7, 127.5, 125.7, 124.2, 123.3, 113.5, 111.5, 70.9, 70.0, 55.1; MS *m*/*z* (EI+) 397 (M+, 3), 250 (35), 91 (100); HRMS calculated for C₂₅H₁₉NO₄ (M+) 397.1314, found 397.1309.

Second to elute was 2-[(*1S,2S*)-6-(benzyloxy)-1-hydroxy-1,2-dihydronaphthalene-2-yl]isoindoline-1,3-dione **56** (99 mg, 25%) as a white solid; *R*_{f} 0.21 (40% EtOAc in hexane); mp 245 °C; [α]_{D25} = +35 (c= 2.2, CHCl₃); IR (CHCl₃)/cm⁻¹ 3500br, 3025m, 1770w, 1717s, 1380m; ₁H NMR (400 MHz) δ 7.89-7.84 (m, 2H), 7.76-7.72 (m, 2H), 7.49-7.31 (m, 6H), 6.88 (dd, *J* = 8, 2.5, 1 H), 6.77 (d, *J* = 2.5, 1 H), 6.51 (dd, *J* = 10, 3, 1H), 5.91 (dd, *J* = 10, 2.5, 1 H), 5.43 (dd, *J* = 12, 9.5, 1 H), 5.14 (ddd, *J* = 12.5, 3, 2.5, 1 H), 5.09 (s, 2H), 1.85 (d, *J* = 9.5, 1 H); ₁₃C NMR (100 MHz) δ 168.4, 158.7, 136.8, 134.1, 133.7, 132.0, 129.5, 128.6, 128.0, 128.0, 127.5, 127.4, 125.6, 123.4, 113.7, 113.5, 70.9, 70.1, 55.3; MS *m*/*z* (EI+) 397 (M+, 3), 250 (53), 91 (100); HRMS calculated for C₂₅H₁₉NO₄ (M₊) 397.1314, found 397.1309.

The enantioselectivies were determined by chiral HPLC.

1,2-Dihydronapthalene **55** conditions: Chiralcel OD; hexane:*i*PrOH = 85:15; flow = 1 mL/min at RT; retention times = 29.92 min (major using (*S*,*R*)-PPF-*t*Bu₂) and 36.02 min (minor using (*S*,*R*)-PPF-*t*Bu₂).

1,2-Dihydronapthalene **56** conditions: Chiralcel OD; hexane:*i*PrOH = 85:15; flow = 1 mL/min at RT; retention times = 31.39 min (major using (*S*,*R*)-PPF-*t*Bu₂) and 43.68 min (minor using (*S*,*R*)-PPF-*t*Bu₂).

In the above reaction conditions it was obtained a 55% yield of desired 2-[(1*S*,2*S*)-7-(benzyloxy)-1- hydroxy-1,2-dihydronaphthalene-2-yl]isoindoline-1,3-dione **55** with 94% ee and a 25% yield of and 2-[(*1S,2S*)-6-(benzyloxy)-1-hydroxy-1,2-dihydronaphthalene-2-yl]isoindoline-1,3-dione **56** with 99% ee.

## Claims

1. A process for preparing a compound according to formula (I) or (Ia) in a high enantiomeric excess, 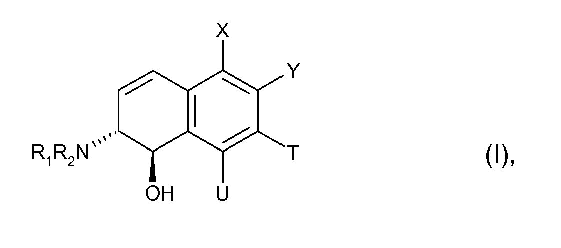 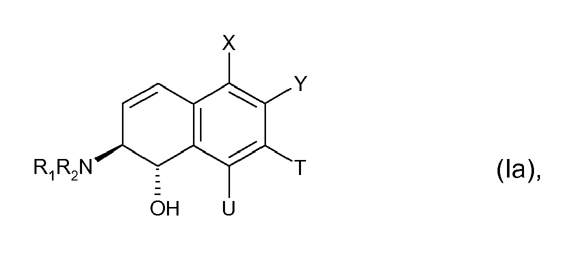 where each of X, Y, U and T is independently selected from the group consisting of H, F, Cl, Br, I, CN, C₁-C₃ alkyl which is possibly substituted by one or more halogen atoms, C₆-C₁₂ aralkyl, C₄-C₈ heteroaralkyl, C₁-C₃ alkoxy, C₆-C₁₂ aralkyloxy, C₁-C₃ alkylthio, an amino group NR₃R₄, a carboxylic acid amido or ester group (CH₂)ₓ-CO-E, x being 1 or 0 and E being OR₅ or NR₆R₇, or a group -OCO-R₅, or X and Y, Y and T, or T and U together represent a C₅-C₈ carbocyclic ring or C₃-C₆ heterocyclic ring containing one or more heteroatoms selected from the group consisting of O, N and S, which may be substituted by one or more C₁-C₃ alkyl, each of R₃ and R₄ is independently selected from the group consisting of C₁-C₁₀ alkyl, C₅-C₈ cycloalkyl, C₆-C₁₂-aralkyl, or R₃ and R₄ together represent -(CH₂)ₙ, n being 3, 4 or 5, -CH₂-CH₂-O-CH₂-CH₂- or -CH₂-CH₂-NR₈-CH₂-CH₂-, R₅ represents C₁-C₁₀ alkyl, C₅-C₈ cycloalkyl or C₆-C₁₂-aralkyl, each of R₆ and R₇ is independently selected from the group consisting of C₁-C₁₀ alkyl, C₅-C₈ cycloalkyl, C₆-C₁₂-aralkyl, or R₆ and R₇ together represent -(CH₂)ₙ, n being 3, 4 or 5, -CH₂-CH₂-O-CH₂-CH₂- or -CH₂-CH₂-NR₈-CH₂-CH₂-, R₈ represents C₁-C₃ alkyl, with the proviso that Y is different from T, and / or X is different from U, and each of R₁ and R₂ is independently selected from the group consisting of H, a C₁-C₁₀ carbon residue which may contain 1 or more heteroatoms selected from O, N and S, or R₁ and R₂ together represent -(CH₂)ₙ, n being 3, 4 or 5, -CH₂-CH₂-O-CH₂-CH₂-, - CH₂-CH₂-NR₈-CH₂-CH₂- or -CO-R₉-CO-, and R₉ represents 1 ,2-phenylene, ethylene, ethenylene, which may be substituted by one or two C₁-C₃ alkyl, the process comprising reacting a nucleophile of formula R₁NHR₂ with a chiral compound of formula (II), in the form of a racemate, 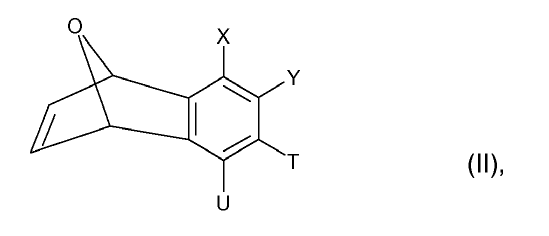 wherein R₁, R₂, X, Y, T and U are defined as above, in the presence of a rhodium-based catalytic system which comprises a chiral diphosphine ligand.

2. The process of claim 1, wherein the chiral diphosphine ligand is selected from the group consisting of (R)-(S)-PPF-P^{t}Bu₂, (S)-(R)-PPF-P^{t}Bu₂, (R)-(S)-BPPFA, (S)-(R)-BPPFA and a chiral analogue of DPPF.

3. The process of claim 1 or 2, wherein the rhodium-based catalytic system produces in situ a Rh(COD)₂ iodide or bromide.

4. The process of any of claims 1 to 3, wherein the nucleophile of formula R₁NHR₂ is selected from the group consisting of phtalimide, piperidine, pyrrolidine, indole, tetrahydroquinoline, dibenzylamine, benzylamine, p-methoxybenzylamine, dipropylamine and propylamine.

5. A compound according to formula (I) or (Ia) in a high enantiomeric excess, 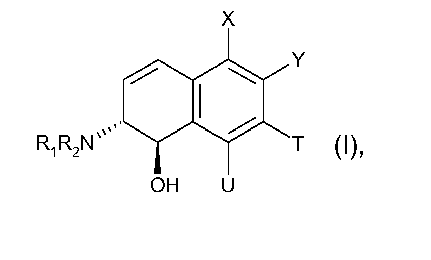 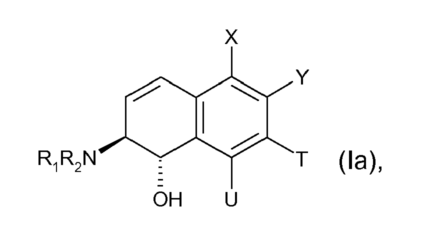 where each of X, Y, U and T is independently selected from the group consisting of H, F, Cl, Br, I, CN, C₁-C₃ alkyl which is possibly substituted by one or more halogen atoms, C₆-C₁₂ aralkyl, C₄-C₈ heteroaralkyl, C₁-C₃ alkoxy C₆-C₁₂ aralkyloxy, C₁-C₃ alkylthio, an amino group NR₃R₄, a carboxylic acid amido or ester group (CH₂)ₓ-CO-E, x being 1 or 0 and E being OR₅ or NR₆R₇, or a group -OCO-R₅, or X and Y, Y and T, or T and U together represent a C₅-C₈ carbocyclic ring or C₃-C₆ heterocyclic ring containing one or more heteroatoms selected from the group consisting of O, N and S, which may be substituted by one or more C₁-C₃ alkyl, each of R₃ and R₄ is independently selected from the group consisting of C₁-C₁₀ alkyl, C₅-C₈ cycloalkyl, C₆-C₁₂-aralkyl, or R₃ and R₄ together represent -(CH₂)ₙ, n being 3, 4 or 5, -CH₂-CH₂-O-CH₂-CH₂- or -CH₂-CH₂-NR₈-CH₂-CH₂-, R₅ represents C₁-C₁₀ alkyl, C₅-C₈ cycloalkyl or C₆-C₁₂₋aralkyl, each of R₆ and R₇ is independently selected from the group consisting of C₁-C₁₀ alkyl, C₅-C₈ cycloalkyl, C₆-C₁₂-aralkyl, or R₆ and R₇ together represent -(CH₂)ₙ, n being 3, 4 or 5, -CH₂-CH₂-O-CH₂-CH₂- or -CH₂-CH₂-NR₈-CH₂-CH₂-, R₈ represents C₁-C₃ alkyl, with the proviso that Y is different from T, and / or X is different from U, and each of R₁ and R₂ is independently selected from the group consisting of H, a C₁-C₁₀ carbon residue which may contain 1 or more heteroatoms selected from O, N and S, or R₁ and R₂ together represent -(CH₂)ₙ, n being 3, 4 or 5, -CH₂-CH₂-O-CH₂-CH₂-, - CH₂-CH₂-NR₈-CH₂-CH₂- or -CO-R₉-CO-, and R₉ represents 1 ,2-phenylene, ethylene, ethenylene, which may be substituted by one or two C₁-C₃ alkyl.

6. The compound of claim 5, wherein one of R₁ and R₂ is H.

7. The compound of claim 6, selected from the group of 2-[(1 S,2S)-5-(benzyloxy) 1-hydroxy-1,2-dihydronaphthalen-2-yl]isoindoline-1,3-dione, 2-[(1S,2S)-7-(benzyloxy) I-hydroxy-1,2-dihydronaphthalen-2-yl]isoindoline-1,3-dione, (1R,2R)-7-(benzyloxy)-2-(benzylisopropylamino)-1,2-dihydronaphthalen-1-ol, (1*R*,2*R*)-2-(dipropylamino)-1,2-dihydro-5-methoxynaphthalen-1-ol, and 2-[(1*S*,2*S*)-7-(benzyloxy)-1,2-dihydro-1-hydroxynaphthalen-2-yl]isoindoline-1,3-dione.

8. Use of the process of any of claims 1 to 4 or the compound of any of claims 5 to 7 for preparing an API selected from the group consisting of:
- (6S)-5,6,7,8-tetrahydro-6-[propyl[2-(2-thienyl)ethyl]amino]-1-naphthalenol,
- [[(7S)-7-[[(2R)-2-(3-chlorophenyl)-2-hydroxyethyl]amino]-5,6,7,8-tetrahydro-2-naphthalenyl]oxy]-acetic acid ethyl ester,
- (4aR,10bR)-3,4,4a,5,6,10b-hexahydro-4-propyl-2H-Naphtho[1,2-b]-1,4-oxazin-9-ol,
- (4aR,10bR)-3,4,4a,5,6,10b-hexahydro-4-propyl-2H-Naphth[1,2-b]-1,4-oxazine-9-carboxamide,
- (S)-1,2,3,4-tetrahydro-8-(methylthio)-N,N-dipropyl-2-naphthalenamine,
- 4-(4-morpholinyl)-N-[(2R)-1,2,3,4-tetrahydro-8-(4-methyl-1-piperazinyl)-2-naphthalenyl]-benzamide,
- (1S,2R)-1,2,3,4-tetrahydro-5-methoxy-1-methyl-N,N-dipropyl-2-naphthalenamine,
- [R-(R,S)]-N,N-dimethyl-2-[[5,6,7,8-tetrahydro-7-[[2-hydroxy-2-(4-hydroxyphenyl)ethyl]amino]-2-naphthalenyl]oxy]-acetamide,
- N,N-dimethyl-2-[[(7S)-5,6,7,8-tetrahydro-7-[[(2R)-2-hydroxy-2-[4-hydroxy-3-(2-hydroxyethyl)phenyl]ethyl]amino]-2-naphthalenyl]oxy]-acetamide,
- 4-(4-morpholinyl)-N-[(2R)-1,2,3,4-tetrahydro-5-methyl-8-(4-methyl-1-piperazinyl)-2-naphthalenyl]-benzamide,
- 6-[[(4-chlorophenyl)sulfonyl]amino]-5,6,7,8-tetrahydro-2-methyl-1-naphthalenepropanoic acid,
- 5,6,7,8-tetrahydro-6-(methylamino)-1,2-naphthalenediol, and
- 2-methyl-propanoic acid 1,1'-[5,6,7,8-tetrahydro-6-(methylamino)-1,2-naphthalenediyl] ester.

9. The process of claim 1 for preparing 2-[(1S,2S)-5-(benzyloxy)-1-hydroxy-1,2-dihydronaphthalen-2-yl]isoindoline-I_{/}3-dione in high enantiomeric excess, wherein the nucleophile of formula R₁NHR₂ is phtalimide.

10. The process of claim 9, wherein 2-[(1S,2S)-5-(benzyloxy)-1-hydroxy-1,2-dihydronaphthalen-2-yl]isoindoline-I_{/}3-dione in high enantiomeric excess is isolated by fractional crystallisation.
